# FASCICULE DE BREVET EUROPEEN

(11) **EP 1 451 573 B1**
(45) Date de publication et mention de la délivrance du brevet: **18.01.2006**
(21) Numéro de dépôt: 02796892.4
(22) Date de dépôt: 03.12.2002
(51) Int. Cl.: G01N 33/02, G01N 21/64

(54) **PROCEDE ET DISPOSITIF DE MESURE DE LA TENDRETE DE LA VIANDE ANIMALE OU DE LA FRAICHEUR DU POISSON**
VORRICHTUNG UND VERFAHREN UM DIE ZARTHEIT VON FLEISCH UND DIE FRISCHHEIT VON FISCH ZU MESSEN
METHOD AND DEVICE FOR MEASURING ANIMAL MEAT TENDERNESS OR FISH FRESHNESS

(30) Priorité: 07.12.2001 FR 0116067
(43) Date de publication de la demande: 01.09.2004
(73) Titulaire: Adiv Association, 63039 Clermont Ferrand cedex2 (FR); Ofival - Office National Interprofessionnel des Viandes de l'Elevage et de l'Aviculture, 75607 Paris (FR); Interbev - Association Nationale Interprofessionelle du Betail et des Viandes, 75595 Paris cedex 12 (FR)
(72) Inventeur: FRENCIA, Jean-Pierre, F-63100 Clermont-Ferrand (FR); DUFOUR, Eric, F-63800 Cournon d'Auvergne (FR)
(74) Mandataire: Dupuis, François
(86) Numéro de dépôt international: PCT/FR2002/004140
(87) Numéro de publication internationale: WO 2003/048761

(56) Documents cités:
- DE-A- 10 013 992
- US-A- 3 067 328
- US-A- 5 918 190
- US-A- 6 088 114
- SWATLAND H J: "CORRECTION FOR BASE-LINE DRIFTING IN PROBE MEASUREMENTS OF CONNECTIVE TISSUE IN BEEF" FOOD RESEARCH INTERNATIONAL, ELSEVIER APPLIED SCIENCE, BARKING, GB, vol. 26, 1993, pages 371-374, XP002063348 ISSN: 0963-9969
- SWATLAND H J ET AL: "UV FIBER-OPTIC PROBE MEASUREMENTS OF CONNECTIVE TISSUE IN BEEF CORRELATED WITH TASTE PANEL SCORES FOR CHEWINESS" FOOD RESEARCH INTERNATIONAL, ELSEVIER APPLIED SCIENCE, BARKING, GB, vol. 28, no. 1, 1995, pages 23-30, XP002063349 ISSN: 0963-9969

## Description

L'invention se rattache au secteur technique de l'industrie de la viande animale ou du poisson.

La qualité et la sécurité alimentaire sont des critères de plus en plus importants pour le consommateur, face aux différentes crises qui se sont propagées à travers le monde ces dernières années avec notamment l'encéphalopathie spongiforme bovine. La notion de qualité fait référence à plusieurs aspects qui peuvent être nutritionnels, sanitaires, technologiques, ou organo-leptiques. Ainsi, selon qu'il s'agit de l'abatteur, du découpeur ou du distributeur, ou du consommateur, la qualité perçue de la viande ou du poisson ne sera pas la même pour tous.

Ainsi, dans le cadre de la sécurité alimentaire, un critère particulièrement important pour le consommateur est l'aspect organo-leptique et plus particulièrement la tendreté de la viande ou la fraîcheur du poisson qui reste un facteur d'achat essentiel et de fidélisation de la clientèle.

Cette propriété présente un caractère critique dans le cas des viandes de bovins pour lesquels la différence de prix entre les muscles dépend, à peu près, exclusivement de leur aptitude à donner une viande tendre après cuisson rapide.

Le problème majeur actuel des industries de la viande est qu'elles ne disposent d'aucune méthode fiable et rapide pour garantir la tendreté de la viande au moment de sa commercialisation.

Le facteur « tendreté » fait l'objet pourtant de nombreuses études et recherches en laboratoire qui ne sont pas passées, à la connaissance du demandeur, à une phase d'utilisation industrielle directement par les entreprises concernées.

La tendreté de la viande dépend de deux composantes :
- la composante collagénique,
- la composante myofibrillaire.

La première représente la dureté de base de la viande et ne varie pas avec la maturation car celle-ci n'a aucun effet sur la quantité ou le degré de réticulation du collagène.

La seconde est la composante myofibrillaire, c'est-à-dire que la tendreté dépend de l'état de contraction des fibres musculaires au moment où la viande « caille » après abattage, ainsi que de la durée de maturation.

Différentes études et techniques ont ainsi été développées.

Un premier axe de recherche relève de techniques mécaniques avec les mesures texturométriques. Cela consiste à prélever un échantillon de viande, après abattage, de quelques centimètres, et de soumettre cet échantillon à des contraintes mécaniques de compression et/ou de cisaillement. La mesure de la force de compression ou cisaillement suggère un indice de tendreté de la viande. La méthode WARNER-BRATZEL consiste à couper l'échantillon à l'aide d'une guillotine et à mesurer la force nécessaire à la découpe du morceau de viande.

On utilise, selon une autre méthode, un appareil dénommé « tendromètre ARMOR » consistant en une série d'aiguilles que l'on enfonce dans les muscles de viande et on mesure la force de pénétration. Cette technique est également limitée à un usage en laboratoire.

Ces différentes méthodes sont utilisées en laboratoire et non applicables industriellement car elles sont lourdes à mettre en oeuvre et nécessitent des appareillages de laboratoire.

D'autres axes de recherche ont été développés à travers des méthodes biochimiques. On mesure notamment la quantité de collagène. Cette technique est à usage essentiellement en laboratoire et est très longue à mettre en oeuvre.

On a développé également, selon l'art antérieur, des mesures par impédance métrie. Dans ce procédé, on mesure l'impédance d'un morceau de viande et, à partir des mesures, on en déduit les caractéristiques de tendreté. Cependant, cette mesure reste très aléatoire car la viande est anisotropie. Cette technique n'est donc pas utilisable à grande échelle.

On a développé d'autres techniques de mesure de la tendreté, et en particulier par ultra-sons. On mesure ainsi la vitesse des ultra-sons en pénétration dans le morceau de viande, ou de l'écho en résultant. Mais cette méthode est sensible à la teneur en Matières Grasses des viandes qui peut varier du simple au triple.

Un autre axe de recherche a été d'utiliser la spectroscopie par infrarouge. Le problème dans ce cas réside aussi en ce que les rayons infrarouge sont sensibles à la matière grasse et les mesures varient d'une manière importante car, comme indiqué précédemment, la matière grasse peut varier dans des proportions de deux à six pour cent, par exemple dans le faux filet de viande.

Très récemment, des recherches ont été menées par la technique de spectroscopie de fluorescence. Cela consiste à envoyer un rayon ultra-violet dans une longueur d'onde déterminée qui provoque l'excitation de différents fluorophores qui deviennent fluorescents. On mesure alors la lumière émise en fonction de la longueur d'onde de fluorescence. Les informations obtenues associées à un traitement mathématique adapté permettent de mesurer la tendreté de la viande. Cette technique est essentiellement développée en laboratoire à partir de morceaux d'échantillon prélevés. US 5918190 décrit une technique similaire pour mésurer la tendreté de la viande. Son inconvenient est d'être invasive.

Un problème similaire se pose également dans l'industrie de la pêche avec la mesure de la fraîcheur du poisson. Dans ce domaine, le phénomène est accentué encore par la plus grande rapidité des pertes de qualité de fraîcheur de ces derniers. US 3067328 décrit une méthode pour mésurer la fraîcheur de crustacés. Les crustacés sont illuminés par une lumière de 250-375 nm. Son inconvenient est la détection visuelle. L.Munck et al, Chemometrics and Intelligent Laboratory Systems 44 (1998), pages 31-60 décrit un procédé de mesure de la qualité de sucre qui met en oeuvre une mésure par spectoscopie. Le sucre doit être mis en solution.

L'ensemble des méthodes ainsi décrites à titre de rappel ne sont pas directement utilisables en sites industriels où les mesures sont excessivement nombreuses dans un laps de temps court puisqu'il est nécessaire de procéder à des tests sur chaque viande animale abattue ou pêchée en un ou plusieurs endroits.

La méthode la plus fiable actuellement reste la méthode sensorielle qui consiste à choisir des individus formés à tester les produits et les évaluer. Cette méthode reste néanmoins ponctuelle, très coûteuse, de part les charges résultant des individus testeurs. Elle est également lente car il n'est pas envisageable de tester des grandes quantités de produits en temps limité.

La démarche du demandeur a donc été de rechercher la mise au point d'un procédé apte à permettre, dans des conditions d'utilisation industrielle, la mesure de la tendreté de la viande animale ou de la fraîcheur du poisson, par un examen de leur chair, d'une manière non invasive (sans nécessiter le prélèvement d'échantillon) et d'une interprétation rapide des résultats de mesure.

Ainsi, selon une première caractéristique de l'invention, le procédé est remarquable en ce qu'il met en oeuvre une mesure de la tendreté de la viande animale ou de la fraîcheur du poisson par spectroscopie de fluorescence, ladite mesure étant établie d'une manière non invasive à partir d'une source lumineuse ultra violet projetée sur une zone spécifique de viande, à mesurer le spectre de fluorescence émis par des fluorophores de ladite zone examinée, ledit spectre étant envoyé dans un spectromètre relié à un ordinateur incluant un système d'acquisition et de traitement de données, apte à traiter ledit spectre pour en déduire, par un traitement mathématique adapté, des paramètres représentatifs de la tendreté ou de la fraîcheur.

Selon une autre caractéristique, les systèmes d'acquisition et de traitement des données permettent l'utilisation de méthodes mathématiques adaptées afin d'extraire l'information pertinente des spectres de fluorescence, et en ce que ces méthodes mathématiques consistent notamment en l'utilisation de méthodes d'analyse statistique multidimensionnelle telles que l'Analyse en Composantes Principales (ACP), l'Analyse Factorielle Discriminante (AFD) et l'Analyse des Corrélations Canoniques, qui permettent de discriminer les échantillons en fonction de la tendreté de la viande ou de la fraîcheur du poisson. Ces méthodes, grâce à une base de données initiale, permettent de classer les échantillons en fonction de leur tendreté ou de leur fraîcheur.

Selon une autre caractéristique, l'énergie lumineuse d'excitation des fluorophores est établie sur une longueur d'onde comprise entre 250 et 290 nm correspondant à la longueur d'onde d'excitation de différents flurophores et notamment des tryptophanes des protéines, des Acides Aminés Aromatiques et des Acides Nucléiques.

Selon une autre caractéristique, l'énergie lumineuse d'excitation est transmise par une fibre optique co-axiale permettant, d'une part, l'excitation des fluorophores et, d'autre part, la réflexion du spectre de fluorescence.

Selon une autre caractéristique, le dispositif est remarquable en ce qu'il comprend, sous forme d'un appareil modulaire et portable, une source lumineuse incluant un filtre interférentiel relié à, au moins, une fibre optique co-axiale assurant la transmission d'une énergie lumineuse sur une zone spécifique de viande, engendrant la formation d'un spectre de fluorescence retransmis à destination d'un spectromètre, ce dernier étant en liaison directe avec un système d'acquisition et de traitement de données apte à traiter le spectre pour en déduire les paramètres représentatifs de la tendreté de la viande ou de la fraîcheur du poisson.

Selon une autre caractéristique, la fibre optique est co-axiale dont une partie assure la transmission de la longueur d'onde d'excitation vers l'échantillon et l'autre la collecte du spectre de fluorescence vers le spectromètre.

Ces caractéristiques et d'autres encore ressortiront bien de la suite de la description.

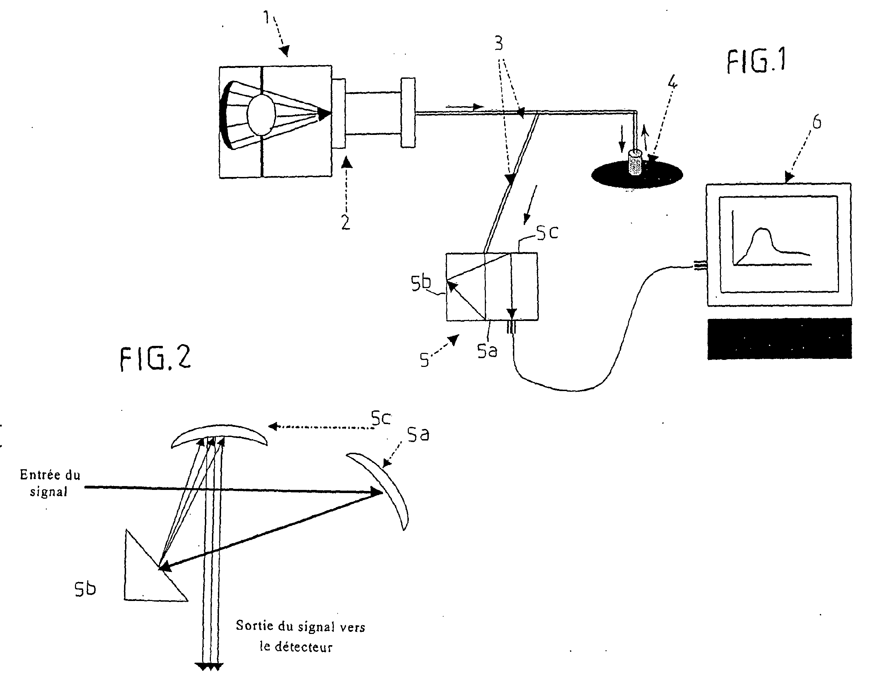

- La figure 1 est une vue du dispositif, selon l'invention, représenté sous forme schématique, le dispositif étant inclus dans un appareil modulaire transportable,
- La figure 2 est une vue du spectromètre intervenant dans le dispositif précité,
- La figure 3 est un exemple de courbe de mesure établie à partir des mesures effectuées avec, en abscisses, la longueur d'onde nm et, en ordonnées, l'intensité de fluorescence,
- La figure 4 est une vue représentant les différentes phases d'analyse des corrélations canoniques, selon un graphe de régression entre un groupe de variables X en abscisses, et un groupe de variable Y en ordonnées.

Afin de rendre plus concret l'objet de l'invention, on le décrit maintenant d'une manière non limitative illustrée aux figures des dessins.

Le dispositif de mesure de spectroscopie de fluorescence, selon l'invention, inclut, dans un appareil modulaire portable, un premier moyen (1) permettant l'émission d'une source lumineuse et incluant un filtre interférentiel (2). Ce premier moyen envoie une longueur d'onde d'excitation dans une fibre optique (3) co-axiale vers une zone (4) spécifique de viande non découpée et non invasive. L'action de la fibre optique sur les fluorophores situées dans la zone précitée permet l'émission d'un spectre de fluorescence qui est renvoyé lui-même par le biais de la fibre optique co-axiale dans un spectromètre (5). Ce dernier comprend intérieurement une pluralité de miroirs (5a - 5b - 5c) permettant une sortie de signal vers un ordinateur (6) incluant des logiciels d'acquisition et de traitement des données, et incluant notamment une méthode d'analyse canonique de corrélation.

Selon une caractéristique particulière, la longueur d'onde de la fibre optique est établie dans une valeur de 250 à 290 nm, c'est-à-dire correspondant à l'excitation de différents fluorophores pour l'obtention d'un effet de spectre par fluorescence.

Plus spécifiquement, cette longueur d'onde d'excitation retenue correspond aux longueurs d'onde d'excitation des tryptophanes, des Acides Nucléiques et des Acides Aminés Aromatiques. Ces longueurs d'onde présentent plusieurs avantages :
- elles se situent dans le domaine des ultra-violets,
- le spectre de fluorescence obtenu discrimine les muscles en fonction de leur dureté de base et de la durée de maturation. Les résultats obtenus avec le spectre de fluorescence du collagène seul a une intensité beaucoup plus faible et un rapport signal/bruit plus défavorable qu'il ne permet pas de discriminer les muscles.
- Le spectre d'émission des protéines présente un maximum pour une longueur d'ondes de 315 à 320 nm qui correspond également aux longueurs d'ondes d'excitation du collagène. Ainsi la fluorescence des protéines myofibrillaires provoque une excitation indirecte de la fluorescence du collagène permettant d'avoir les deux informations simultanément.

La fibre optique est appliquée sur la zone spécifique de viande. Les mesures de spectroscopie de fluorescence sont réalisées préférentiellement en trois essais, en trois endroits différents sur le muscle.

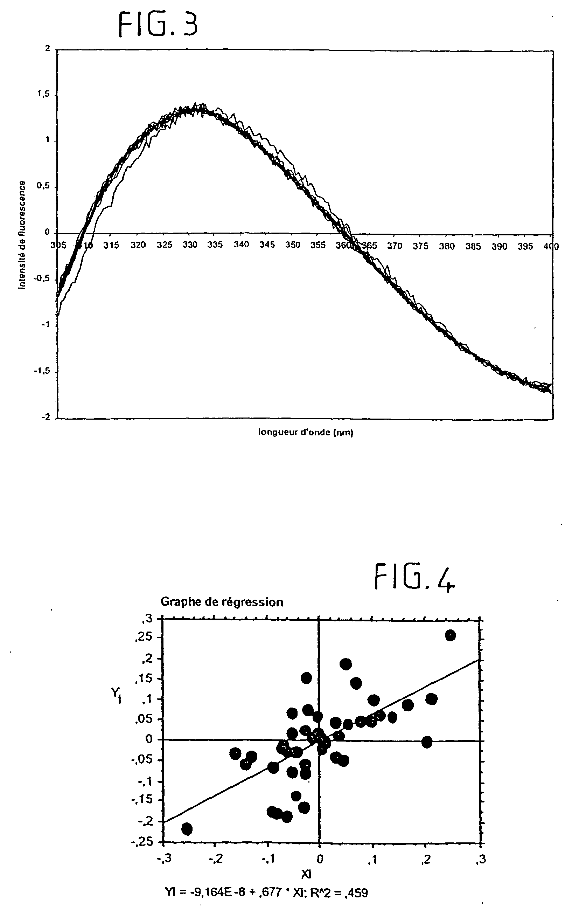

Chaque mesure effectuée entraîne l'émission d'un graphe par le spectre de fluorescence frontal (figure 4) et permettant ainsi une comparaison générale par superposition des graphes lors de mesures successives.

Les différents logiciels d'acquisition de traitement de données permettent alors d'évaluer les résultats. On utilise, de préférence, des méthodes d'analyse statistique multidimensionnelles telles que l'Analyse en Composantes Principales, l'Analyse Factorielle Discriminante et l'Analyse des Corrélations Canoniques.

Le dispositif, selon l'invention, est mis en oeuvre dans le cadre d'une unité portable et il permet ainsi par un opérateur de procéder in situ à des mesures en situation non invasive. Les mesures sont excessivement rapides, une à deux secondes chacune, avec un traitement immédiat par l'ordinateur. Le dispositif, selon l'invention, répond donc aux exigences industrielles d'utilisation.

Le procédé et le dispositif, ainsi décrits selon l'invention, ont fait l'objet de tests de validation comparativement aux méthodes antérieures utilisées avec un texturomètre et mettant en oeuvre la fibre optique en laboratoire. Les résultats de l'invention ont permis de valider la fiabilité du procédé nouveau et du dispositif s'y rapportant.

Sans sortir du cadre de l'invention, l'énergie lumineuse est transmise par deux fibres optiques permettant l'acquisition du spectre de fluorescence, son acheminement vers le spectomètre et sa mesure.

## Revendications

1. Procédé de mesure de la tendreté de la viande ou de la fraîcheur du poisson qui met en oeuvre une mesure par spectroscopie de fluorescence frontale, ladite mesure étant établie d'une manière non invasive à partir d'une source lumineuse projetée sur une zone spécifique de viande, à mesurer le spectre de fluorescence émis par des fluorophores de ladite zone examinée, ledit spectre étant adressé dans un spectromètre (5) relié à un ordinateur (6) incluant un système d'acquisition et de traitement mathématique des données, apte à traiter ledit spectre pour en déduire des paramètres représentatifs de la tendreté de la viande ou de la fraîcheur du poisson,
et en ce que l'énergie lumineuse d'excitation des fluorophores est établie sur une longueur d'onde de 250 à 290 nm correspondant à la longueur d'onde d'excitation des fluorophores et notamment des tryptophanes des protéines, des Acides Aminés Aromatiques et des Acides Nucléiques.

2. Procédé, selon la revendication 1, **caractérisé en ce que** les systèmes d'acquisition et de traitement des données permettent l'utilisation de méthodes d'analyse statistique multidimensionnelle telles que l'Analyse en Composantes Principales, l'Analyse Factorielle Discriminante et l'Analyse des Corrélations Canoniques.

3. Procédé, selon la revendication 1, **caractérisé en ce que** l'énergie lumineuse est transmise par une fibre optique (3) co-axiale permettant, d'une part, l'excitation des fluorophores et, d'autre part, l'acheminement du spectre de fluorescence vers le spectromètre.

4. Procédé, selon la revendication 1, **caractérisé en ce que** l'énergie lumineuse est transmise par deux fibres optiques permettant l'acquisition du spectre de fluorescence, son acheminement vers le spectromètre et sa mesure.

## Patentansprüche

1. Verfahren, um die Zartheit von Fleisch und die Frischheit von Fisch zu messen, bei dem eine Messung durch Spektroskopie mit Frontalfluoreszenz verwendet wird, wobei die Messung nicht invasiv, ausgehend von einer Lichtquelle durchgeführt wird, welche auf einen bestimmten Bereich des Fleisches projiziert wird, um das Fluoreszenzspektrum zu messen, das von den Fluorophoren des untersuchten Bereichs ausgesendet wird, wobei das Spektrum in einem Spektrometer (5) angesprochen wird, der mit einem Computer (6) verbunden ist und ein System der Erfassung und mathematischen Verarbeitung von Daten besitzt, und das Spektrum verarbeiten kann, um daraus repräsentative Parameter für die Zartheit von Fleisch und Frischheit von Fisch abzuleiten, und Lichtenergie für die Erregung von Fluorophoren über eine Wellenlänge von 250 bis 290 nm, entsprechend der Wellenlänge der Erregung der Fluorophore, und insbesondere der Tryptophane der Proteine, der aromatischen Aminosäuren und der Nukelinsäuren erzeugt wird.

2. Verfahren nach Anspruch 1, **dadurch gekennzeichnet, dass** die Datenerfassungs- und verarbeitungssysteme die Verwendung von Methoden der multidimensionalen, statistischen Analyse erlauben, wie die Hauptkomponenten-Analyse, die Faktor-Diskriminanz-Analyse und die kanonische Korrelations-Analyse.

3. Verfahren nach Anspruch 1, **dadurch gekennzeichnet, dass** die Lichtenergie durch eine koaxiale, optische Faser (3) übertragen wird, die einerseits die Erregung der Fluorophore und andererseits die Weiterleitung des Fluoreszenzspektrums zu dem Spektrometer erlaubt.

4. Verfahren nach Anspruch 1, **dadurch gekennzeichnet, dass** die Lichtenergie durch zwei optische Fasern übertragen wird, welche die Erfassung des Fluoreszenzspektrums, seine Weiterleitung zu dem Spektrometer und seine Messung erlauben.

## Claims

1. Procedure for measuring the tenderness of meat or the freshness of fish which uses measurement by frontal fluorescence spectroscopy, said measurement being made in a non-invasive manner using a light source projected onto a specific area of meat, and which measures the fluorescence spectrum emitted by fluorophores in said examined area, said spectrum being addressed in a spectrometer (5) linked to a computer (6) including a data acquisition and mathematical processing system capable of processing said spectrum in order to deduce parameters representative of the tenderness of meat or the freshness of fish from it,
and the luminous energy used to excite the fluorophores has a wavelength of 250 to 290 nm equivalent to the excitation wavelength of the fluorophores and especially the tryptophans of proteins, Aromatic Amino Acids and Nucleic Acids.

2. A procedure as claimed in claim 1, **characterised in that** the data acquisition and processing systems allow the use of multidimensional statistical analysis methods such as Principal Component Analysis, Discriminant Factorial Analysis and Canonical Correlation Analysis.

3. A procedure as claimed in claim 1, **characterised in that** the luminous energy is transmitted by a coaxial optical fibre cable (3) allowing, firstly, excitation of the fluorophores and, secondly, routing of the fluorescence spectrum to the spectrometer.

4. A procedure as claimed in claim 1, **characterised in that** the luminous energy is transmitted by two optical fibre cables allowing acquisition of the fluorescence spectrum, its routing to the spectrometer and its measurement.
